# EUROPEAN PATENT APPLICATION

(11) **EP 4 140 469 A1**
(43) Date of publication of application: **01.03.2023**
(21) Application number: 20931879.9
(22) Date of filing: 21.05.2020
(51) Int. Cl.: A61K 8/34, A61Q 19/00, C12P 7/18

(54) **DISPERSANT OR DISPERSION MEDIA COMPOSITION COMPRISING 2,3-BUTANEDIOL, AND COSMETIC COMPOSITION COMPRISING SAME**

(30) Priority: 22.04.2020 KR 20200048969
(71) Applicant: GS Caltex Corporation, Seoul 06141 (KR)
(72) Inventor: CHOI, Seng-Hye, Daejeon 34048 (KR); PARK, Jong-Myoung, Sejong-si 30124 (KR)
(74) Representative: Zacco Sweden AB
(86) International application number: PCT/KR2020/006658
(87) International publication number: WO 2021/215574

(57) **Abstract**

The present invention relates to a dispersant composition comprising 2,3-butanediol, and to a cosmetic composition comprising the dispersant composition. In addition, the present invention relates to a dispersion media composition comprising 2,3-butanediol, and to a cosmetic composition comprising the dispersion media composition.

## Description

### FIELD

The present disclosure relates to a dispersant or dispersion medium composition comprising 2,3-butanediol. Further, the present disclosure relates to a cosmetic composition comprising the dispersant or dispersion medium composition.

### DESCRIPTION OF RELATED ART

In the human body, the skin is a part in contact with the external environment and is responsible for protecting the inside of the human body from various factors from the external environment. Traditionally, cosmetics have been used to improve and preserve skin condition, and various raw materials are being developed as components of cosmetics.

For example, Korean Patent No. 10-1914844 describes a cosmetic composition comprising niacinamide and ricinoleate compounds. Korean Patent No. 10-0746058 describes a self-tanning cosmetic composition comprising a vitamin B3 compound. Korean Patent No. 10-0292143 describes a cosmetic composition comprising glycolic acid and vitamin C.

However, in order to effectively distribute and use the cosmetics, it is important to keep the cosmetics in a stable manner. Inventors of the present disclosure were working on dispersant and dispersion medium for use in a cosmetic composition. Thus, we identified that when a specific isomer of 2,3-butanediol was used, vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients were uniformly dispersed in the cosmetic composition, and the cosmetic composition was kept in a stable manner. Thus, the present disclosure has been completed.

### DISCLOSURE

### TECHNICAL PURPOSE

A purpose of the present disclosure is to provide an effective dispersant composition and dispersion medium composition for use in cosmetic compositions.

### TECHNICAL SOLUTION

In order to achieve the above purpose, the present disclosure provides a dispersant composition comprising 2,3-butanediol.

Further, the present disclosure provides a cosmetic composition comprising the dispersant composition.

Further, the present disclosure provides a dispersion medium composition comprising 2,3-butanediol.

Further, the present disclosure provides a cosmetic composition comprising the dispersion medium composition.

Further, the present disclosure provides a method for producing a dispersant composition, the method comprising adding and mixing 2,3-butanediol to and with a solvent.

Further, the present disclosure provides a method for producing a cosmetic composition, the method comprising adding and mixing the dispersant composition produced using the producing method according to the present disclosure to and with a cosmetic material.

Further, the present disclosure provides a method of producing a dispersion medium composition, the method comprising adding and mixing 2,3-butanediol to and with a solvent.

Further, the present disclosure provides a method for producing a cosmetic composition, the method comprising adding and mixing the dispersion medium composition produced by the producing method according to the present disclosure to and with a cosmetic material.

### TECHNICAL EFFECT

In accordance with the present disclosure, the dispersant composition and/or dispersion medium composition may be used so as to uniformly disperse certain active ingredients in the cosmetic composition and keep the cosmetic composition in a stable manner.

### BRIEF DESCRIPTION OF DRAWINGS

FIG. 1 shows a 100% eco-friendly bio-process using microbial fermentation as a process in which 2,3-butanediol according to the present disclosure is produced.
FIG. 2 shows a dispersion mechanism indicating that a dispersion force is determined based on physical characteristics such as a freezing point and a molecular structure of 2,3-butanediol.
FIG. 3 shows Hansen solubility parameter values of various polyols.
FIG. 4 is a photograph evaluating a dispersion force of allantoin after storing a liquid mixture of each of Production Examples 1 to 4 under a refrigeration (4°C) condition in water for 7 days.
FIG. 5 is a photograph evaluating a dispersion force of allantoin after storing a liquid mixture of each of Production Examples 5 to 8 under a refrigeration (4°C) condition in a solubilized formulation for 7 days.
FIG. 6 is a photograph after storing each of P/O emulsions of Production Examples 9 to 12 for 8 weeks.
FIG. 7 is a photograph after storing each of P/O emulsions of Production Examples 13 to 16 for 8 weeks.
FIG. 8 is a photograph evaluating a dispersion force of ceramide after storing a liquid mixture of each of Production Examples 17 to 20 for 1 day at room temperature.
FIG. 9 is a photograph evaluating a dispersion force of ceramide after storing a liquid mixture of each of Production Examples 17 to 20 at room temperature for 4 weeks.
FIG. 10 is a photograph evaluating a dispersion force of ceramide after storing a liquid mixture of each of Production Examples 17 and 19 under a thermostat (45°C) condition for 4 weeks.
FIG. 11 is a photograph evaluating a dispersion force of ceramide after storing a liquid mixture of each of Production Examples 21 to 24 under a room temperature condition for 1 day in a solubilized skin formulation.
FIG. 12 is a photograph evaluating a dispersion force of ceramide after storing a liquid mixture of each of Production Examples 21 to 24 under a room temperature condition for 4 weeks in a solubilized skin formulation.

### BEST MODE FOR IMPLEMENTING PRESENT DISCLOSURE

The present disclosure provides a dispersant composition comprising 2,3-butanediol.

Further, the present disclosure provides a cosmetic composition comprising the dispersant composition.

Further, the present disclosure provides a dispersion medium composition comprising 2,3-butanediol.

Further, the present disclosure provides a cosmetic composition comprising the dispersion medium composition.

Further, the present disclosure provides a method for producing a dispersant composition, the method comprising adding and mixing 2,3-butanediol to and with a solvent.

Further, the present disclosure provides a method for producing a cosmetic composition, the method comprising adding and mixing the dispersant composition produced using the producing method according to the present disclosure to and with a cosmetic material.

Further, the present disclosure provides a method of producing a dispersion medium composition, the method comprising adding and mixing 2,3-butanediol to and with a solvent.

Further, the present disclosure provides a method for producing a cosmetic composition, the method comprising adding and mixing the dispersion medium composition produced by the producing method according to the present disclosure to and with a cosmetic material.

Hereinafter, the present disclosure will be described in detail.

### Dispersant composition and/or dispersion medium composition and method for producing the same

The present disclosure relates to a dispersant and/or dispersion medium composition comprising 2,3-butanediol. Further, the present disclosure relates to a method for producing a dispersant and/or dispersion medium composition, the method comprising adding and mixing 2,3-butanediol to and with a solvent. The 2,3-butanediol may be (2R,3S)-2,3-butanediol. The (2R,3S)-2,3-butanediol according to the present disclosure may include 2,3-butanediol isomers, wherein a content of a (2R,3S)-2,3-butanediol isomer may be the greatest. For example, (2R,3S)-2,3-butanediol according to the present disclosure may comprise (2R,3S)-2,3-butanediol in a larger amount than that of (2R,3R)-2,3-butanediol, or may comprise (2R,3S)-2,3-butanediol in a larger amount than that of (2S,3 S)-2,3-butanediol. Preferably, the (2R,3S)-2,3-butanediol may be a composition comprising 2,3-butanediol isomers, wherein in the composition, a content of (2R,3 S)-2,3-butanediol may be greater than a sum of contents of 2,3-butanediol isomers other than the (2R,3S)-2,3-butanediol. Preferably, the (2R,3S)-2,3-butanediol according to the present disclosure may be a mixture of 2,3-butanediol isomers in which a content of (2R,3S)-2,3-butanediol is 80% by weight or greater.

The solvent may be any solvent that may be used for general dispersant/dispersion medium composition, and is not particularly limited. For example, the solvent may be a synthetic solvent or a natural solvent, and may be water, an organic solvent, etc., for example, water, ethanol, alcohols including a polyol having 2 to 8 carbon atoms, oil, or the like.

The dispersant and/or dispersion medium composition according to the present disclosure may comprise 2,3-butanediol according to the present disclosure. In this regard, the 2,3-butanediol according to the present disclosure may be contained at a content of 0.1 to 100% by weight, preferably 0.2 to 99% by weight, based on 100% by weight of the dispersant and/or dispersion medium composition.

In 2,3-butanediol, -OH groups are respectively located at second and third carbons and are arranged symmetrically to form a rigid structure. Accordingly, 2,3-butanediol has a unique characteristic that it is crystallized at room temperature. Further, since 2,3-butanediol has a hydrophilic hydroxyl group and a lipophilic carbon chain which are balanced with each other, it combines with a hydrophilic active substance such that the substance is easily dispersed in an oil-soluble substance, or combines with a lipophilic active substance such that the substance is easily dispersed in a hydrophilic substance, and thus serves as a dispersant or dispersion medium. The dispersant/dispersion medium according to the present disclosure is applicable to cosmetic compositions, such as water, solubilized formulations, emulsion type formulations, oil-dispersed formulations, and the like.

The dispersant and/or dispersion medium composition according to the present disclosure may provide excellent dispersion force of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients. Thus, the above materials and ingredients may be uniformly dispersed in the cosmetic composition and thus the cosmetic composition may be kept in a stable manner. The above materials and ingredients must be well dispersed when producing the cosmetics. For example, when the temperature is low, allantoin may be precipitated well, thereby causing a problem of precipitation in the cosmetic production. For example, the dispersant and/or dispersion medium composition according to the present disclosure may have the dispersion force of an active ingredient of the cosmetic composition greater than the dispersion force of the active ingredient of the cosmetic composition which (2R,3R)-2,3-butanediol or a 2,3-butanediol isomer mixture with the highest content of (2R,3R)-2,3-butanediol has. In this regard, the active ingredient of the cosmetic composition may include at least one selected from a group consisting of active ingredients including vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients. In this regard, the dispersion force of the at least one selected from a group consisting of active ingredients including vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients may be measured as follows: a sample (that is, the dispersant and/or dispersion medium composition according to the present disclosure) may be mixed with the active ingredient (that is, vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients) to prepare a mixture, and then the mixture may be formulated into a water based formulation or a solubilized formulation, and then the formulation may be stored for 7 days or more, for example, 4 or 8 weeks at a room temperature or 4 °C, and then a stability of the active ingredient may be evaluated and measured based on a precipitation amount of the active ingredient and a phase separation of the oil phase and the aqueous phase. Alternatively, the dispersion force may be measured according to the methods of Experimental Examples <2-1> to <4-2>. The components such as plant extracts, fragrances, and pigments may include plant extracts (e.g. Centella asiatica extract, green tangerine extract, broccoli extract, calendula petal extract, etc.), fragrances, pigments, etc. generally used in the cosmetic composition, and there is no particular limitation thereon. The present disclosure is not limited to the Experimental Example.

2,3-butanediol according to the present disclosure is preferably a biologically based 2,3-butanediol obtained via microbial fermentation. For example, 2,3-butanediol according to the present disclosure may be produced using Bacillus, Enterobacter, Klebsiella, Citrobacter, Escherichia coli, yeast (Saccharomyces), etc. 2,3-butanediol in the present disclosure is not produced using animal-derived raw materials. 2,3-butanediol according to the present disclosure may be a mixture of 2,3-butanediol isomers produced by fermenting microorganisms. There is no artificial treatment for increasing a content of a specific isomer among the 2,3-butanediol isomers after the fermentation. That is, 2,3-butanediol according to the present disclosure may be a mixture of 2,3-butanediol isomers with a high content of (2R,3S)-2,3-butanediol as produced via microbial fermentation. (2R,3S)-2,3-butanediol according to the present disclosure may comprise 2,3-butanediol isomers, wherein a content of (2R,3S)-2,3-butanediol isomer may be the greatest. For example, (2R,3S)-2,3-butanediol according to the present disclosure may comprise (2R,3S)-2,3-butanediol in a larger amount than that of (2R,3R)-2,3-butanediol, or may comprise (2R,3S)-2,3-butanediol in a larger amount than that of (2S,3S)-2,3-butanediol. Preferably, the (2R,3S)-2,3-butanediol may be a composition comprising 2,3-butanediol isomers, wherein in the composition, a content of (2R,3 S)-2,3-butanediol may be greater than a sum of contents of 2,3-butanediol isomers other than the (2R,3S)-2,3-butanediol. Preferably, the (2R,3S)-2,3-butanediol according to the present disclosure may be a mixture of 2,3-butanediol isomers in which a content of (2R,3 S)-2,3-butanediol is 80% by weight or greater. When chemically producing 2,3-butanediol, a chemical catalyst cannot distinguish the isomers from each other, so that the isomers may be produced at random, and a production percentage of (2R,3 S)-2,3-butanediol may be low, which is not the case in 2,3-butanediol according to the present disclosure. In general, 2,3-butanediol isomers are not separated from each other in an easy manner. Thus, when chemically producing 2,3-butanediol, it is difficult to produce (2R,3S)-2,3-butanediol according to the present disclosure, or a mixture of 2,3-butanediol isomers in which a content of (2R,3S)-2 is high according to the present disclosure.

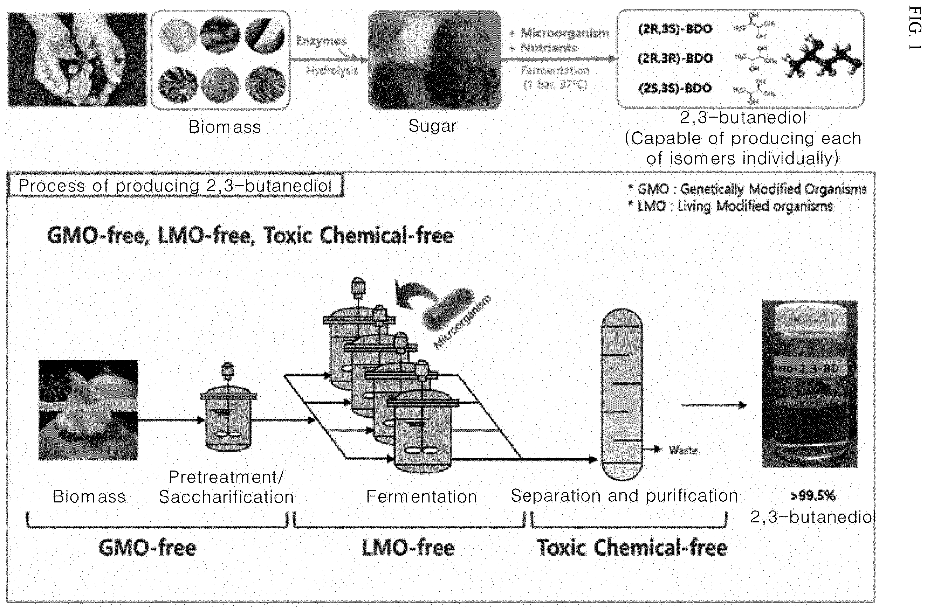

2,3-butanediol according to the present disclosure is produced using a 100% eco-friendly bio process produced via microbial fermentation using biomass as a raw material (FIG. 1). This microorganism was developed by taking samples from nature, such as soil and farms. 2,3-butanediol according to the present disclosure is separated and produced with high purity via a 'fermentation' process in which the microorganisms consume and digest sugar derived from biomass. 2,3-butanediol according to the present disclosure is produced using non-GMO (non-genetically modified) biomass raw materials and microorganisms, and in an eco-friendly manner using only physical characteristics such as boiling point and size during the separation and purification process. When producing 2,3-butanediol according to the present disclosure, the separation and purification process is composed only of filtration, ion removal (electrodialysis, ion exchange), distillation, and decolorization/deodorization (adsorption, distillation) processes. In addition, no animal-derived raw materials were used during the process, and animal tests were not performed on the material.

Unlike the chemical process, the bioprocess has the advantage of being able to produce a specific isomer at a high production percentage. Microorganisms that act as biocatalysts and enzyme proteins constituting the microorganisms have more complex structures than those of chemical catalysts, and thus distinguish the isomers from each other so that the specific isomers may be produced in a specific manner. Therefore, under the bioprocess, the isomer mixture may be produced in which a content of (2R,3S)-2,3-butanediol isomer among isomers (2R,3S)/(2R,3R)/(2S,3S) (in order or meso/levo/dextro) may be the highest. A production percentage of (2R,3S)-2,3-butanediol under the chemical process is low compared to that of (2R,3S)-2,3-butanediol under the bio process. That is, when chemically producing 2,3-butanediol, a chemical catalyst cannot distinguish the isomers from each other, so that the isomers may be produced at random, and a production percentage of (2R,3 S)-2,3-butanediol may be low, which is not the case in 2,3-butanediol according to the present disclosure. In addition, under the chemical process, petroleum-derived hydrocarbons are used as a main raw material, and chemical catalytic reactions of several steps such as chlorohydrination and hydrolysis are performed to produce the 2,3-butanediol. In this case, butene oxide, butane, chlorohydrin, and aldehyde-based chemicals may be produced as by-products. When these substances are applied to cosmetics, they may cause skin irritation or trouble. Therefore, 2,3-butanediol derived from petrochemicals are not employed under megatrend in the cosmetics industry, and are not easily applied to the cosmetics technically. However, 2,3-butanediol produced via the bio-process has been already registered as a food additive as a natural substance. Further, under the bio-process, acetoin, which does not irritate the skin may be produced as by-products and thus may be used as a cosmetic raw material.

2,3-butanediol according to the present disclosure has received global eco-friendly certifications such as COSMOS (Cosmetic Organic Standard) certification as an international certification system for organic cosmetics, 100% bio-product certification by USDA (US Department of Agriculture), and Vegan certification granted to products that do not use animal-derived raw materials and are tested on animals, and thus eco-friendliness and safety thereof have been appreciated.

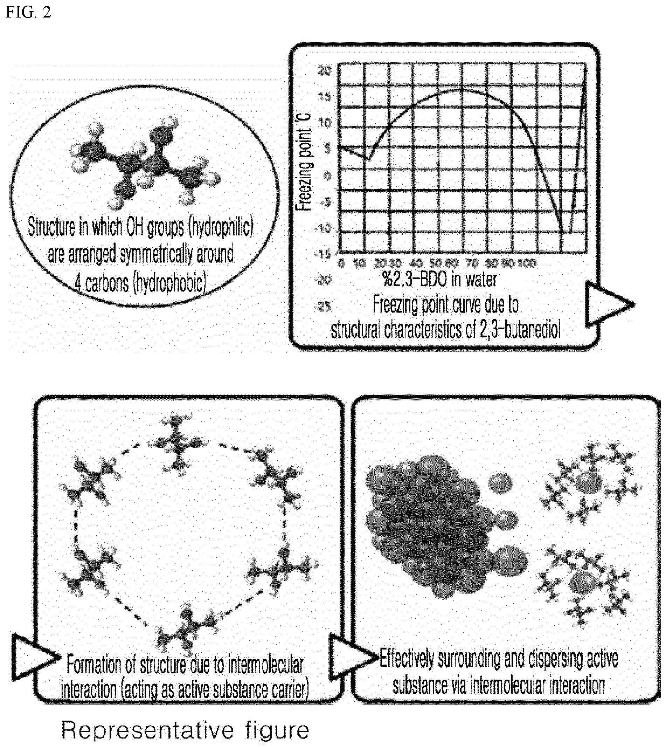

The dispersant and/or dispersion medium composition comprising the biologicallybased 2,3-butanediol according to the present disclosure may disperse vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients in the cosmetic composition more uniformly such that the cosmetic composition is more stably kept than the dispersant and/or dispersion medium composition comprising chemically synthesized 2,3-butanediol may. As shown in FIG. 2, the dispersion force may be determined based on physical characteristics such as the freezing point and molecular structure of 2,3-butanediol. 2,3-butanediol, especially 2,3-butanediol with a high content of (2R,3S)-2,3-butanediol has a high freezing point higher than 13 degrees C. In this regard, being frozen easily may result in easy formation of a specific structure. It is predicted that (2R,3 S)-2,3-butanediol forms a specific structure well because (2R,3S)-2,3-butanediol has a symmetrical structure in which the -OH groups are arranged in a staggered manner. Due to these characteristics of 2,3-butanediol, 2,3-butanediol may easily form the specific structure. When 2,3-butanediol disperses a specific substance, 2,3-butanediol is expected to effectively surround and disperse the substance due to intermolecular interaction. According to the present disclosure, it is identified that 2,3-butanediol has the dispersion force greater than that of each of other polyols such as 1,3-propanediol and 1,3-butylene glycol, and the dispersion force varies based on the type and the composition of isomers of 2,3-butanediol.

### Cosmetic composition and production method thereof

The present disclosure relates to a cosmetic composition comprising the dispersant and/or dispersion medium composition according to the present disclosure. Further, the present disclosure relates to a method for producing a cosmetic composition, the method comprising adding and mixing the dispersant and/or dispersion medium composition produced by the method according to the present disclosure to and with a cosmetic material.

The cosmetic composition according to the present disclosure may be produced into any formulation as commonly produced in the art. The formulation may include solutions, suspensions, emulsions, pastes, gels, creams, lotions, powders, soaps, surfactant-containing cleansing, oils, powder foundations, emulsion foundations, wax foundations and sprays, etc. However, the present disclosure is not limited thereto. Further, the cosmetic composition according to the present disclosure may be produced into a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.

The cosmetic material is not particularly limited as long as it is a cosmetic material generally used in the production of cosmetic compositions. For example, the cosmetic material may include purified water, ethanol, moisturizer, thickener, higher alcohol, emulsifier, auxiliary emulsifier, oil, wax, skin emollient, neutralizer, antioxidant, sequestering agent, preservative, whitening agent, sunscreen, wrinkle suppressing agent, a functional additive, a fragrance, a colorant, an extract, and the like.

The cosmetic composition according to the present disclosure may contain at least one selected from a group consisting of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients.

For example, the cosmetic composition according to the present disclosure may be a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.

The cosmetic composition according to the present disclosure comprises the dispersant and/or dispersion medium composition according to the present disclosure. In this regard, the dispersant and/or dispersion medium composition according to the present disclosure may have the dispersion force of an active ingredient of the cosmetic composition greater than the dispersion force of the active ingredient of the cosmetic composition which (2R,3R)-2,3-butanediol or a 2,3-butanediol isomer mixture with the highest content of (2R,3R)-2,3-butanediol has. In this regard, the active ingredient of the cosmetic composition may include at least one selected from a group consisting of active ingredients including vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients. In this regard, the dispersion force of the at least one selected from a group consisting of active ingredients including vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients may be measured as follows: a sample (that is, the dispersant and/or dispersion medium composition according to the present disclosure) may be mixed with the active ingredient (that is, vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients) to prepare a mixture, and then the mixture may be formulated into a water based formulation or a solubilized formulation, and then the formulation may be stored for 7 days or more, for example, 4 or 8 weeks at a room temperature or 4 °C, and then a stability of the active ingredient may be evaluated and measured based on a precipitation amount of the active ingredient and a phase separation of the oil phase and the aqueous phase. Alternatively, the dispersion force may be measured according to the methods of Experimental Examples <2-1> to <4-2>.

The cosmetic composition according to the present disclosure may comprise the dispersant and/or dispersion medium composition according to the present disclosure, wherein a content of 2,3-butanediol according to the present disclosure comprises may be in a range of 0.5 to 25% by weight, preferably, 5 to 20% by weight, based on 100% by weight of the cosmetic composition.

### EXAMPLES

The benefits and features of the present disclosure, and a method to achieve them will become clear with reference to the Examples as described below in detail. However, the present disclosure will not be limited to the Examples as disclosed below, but will be implemented in a variety of different forms, Only these Examples are provided to ensure that the present disclosure is complete, and to fully inform those of ordinary skill in the art to which the present disclosure belongs, the scope of the disclosure. A scope of the present disclosure is only defined by the claims.

### <Materials and Methods>

PEG-60 hydrogenated castor oil (EMALEX HC 60) was used as a solubilizer.

ELASTOMER BASE which is commercially available and is a mixture of Cyclopentasiloxane, Dimethicone/Vinyl Dimethicone Crosspolymer, Dimethicone, and Phenyl Trimethicone was used as a silicone elastomer.

EMERGENT JC 500B which is commercially available and is a mixture of Cetearyl Ethylhexanoate, Bentone, and Polymethylsilsesquioxane was used as an oil phase stabilizer.

ABIL EM 90 and KF 6017 were used as an emulsifier. ABIL EM 90 is a commercially available product that is a mixture of Cetyl PEG/PPG-10/a Dimethicone, and KF 6017 is a commercially available product that is PEG-10 Dimethicone.

KSG-16 was used as a silicone gel. KSG-16 is a commercially available product that is a mixture of Dimethicone and Dimethicone/Vinyl Dimethicone.

### Present Examples and Comparative Examples

Polyols were prepared as follows. (2S,3S)-2,3-butanediol has similar properties as those of (2R,3R)-2,3-butanediol because -OH groups are located at the same positions in (2S,3S)-2,3-butanediol and (2R,3R)-2,3-butanediol.
Present Examples 1 to 6 are (2R,3 S)-2,3butanediol.
Comparative Example 1 is (2R,3R)-2,3-butanediol.
Comparative Example 2 is 1,3-butylene glycol.
Comparative Example 3 is 1,3-propanediol.
Comparative Example 4 is (2R,3R)-2,3-butanediol.
Comparative Example 5 is 1,3-butylene glycol.
Comparative Example 6 is 1,3-propanediol.
Comparative Example 7 is (2R,3R)-2,3-butanediol.
Comparative Example 8 is 1,3-butylene glycol.
Comparative Example 9 is 1,3-propanediol.
Comparative Example 10 is (2R,3R)-2,3-butanediol.
Comparative Example 11 is 1,3-butylene glycol.
Comparative Example 12 is 1,3-propanediol.
Comparative Example 13 is glycerin.
Comparative Example 14 is 1,3-butylene glycol.
Comparative Example 15 is 1,3-propanediol.
Comparative Example 16 is glycerin.
Comparative Example 17 is 1,3-butylene glycol.
Comparative Example 18 is 1,3-propanediol.

### <Experimental Example 1> Analysis of polyols

The materials were analyzed and compared with each other in terms of factors related to dispersion. This was done by calculating the Hansen solubility parameters.

The Hansen solubility parameter predicts solubility between substances via analysis of the parameters of substances based on the principle of "Like dissolves like". As the Hansen solubility parameter is smaller, similarity between the substances is greater and the substances dissolve each other very well. In this experiment, dispersion-related parameters (Dipole-dipole, hydrogen bond, Dispersion) of the polyols including 2,3-butanediol were evaluated using a HSPiP (Hansen Solubility Parameters in Practice) software.

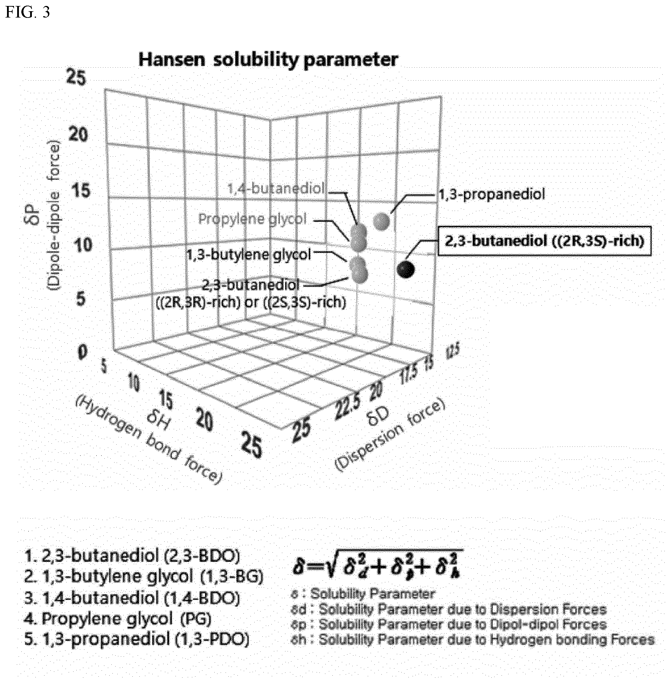

As a result, it was identified that although the materials are similar diols having the same carbon number, each diol having two OH groups, the parameters related to dispersion thereof are different from each other. Therefore, it was predicted that the dispersion forces of the polyols were different from each other (FIG. 3).

### <Experimental Example 2> Evaluation of dispersibility for allantoin

Precipitation of allantoin occurs well when the temperature is low. When allantoin is precipitated during cosmetic production, it is problematic. In accordance with the present disclosure, using the property of allantoin whose precipitation occurs well, the dispersion force was identified in a short time by measuring an amount of precipitation of allantoin based on each of the polyols under a severe refrigeration condition when the temperature is low, and then the dispersion forces of the various polyols were compared with each other. The stability was evaluated at each of a room temperature, a refrigeration temperature, and thermostat (45°C).

### <2-1> Allantoin dispersion force evaluation

The solubility of allantoin is 0.5 g/100 mL. In this experiment, the dispersion force of allantoin, that is, the precipitation amount, and a precipitation rate thereof, based on each of the polyols was evaluated.

### <Production Example 1>

0.02 parts by weight of EDTA-2Na (Disodium Ethylenediaminetetraacetate), 2 parts by weight of 1,2-hexanediol, 0.5 parts by weight of allantoin, and 10 parts by weight of the composition of Present Example 1 were added to 100 parts by weight of de-ionized water to prepare a mixed solution and the mixed solution was stirred at room temperature for 10 minutes.

### <Production Example 2>

The same procedure as that of Production Example 1 was performed except that the polyol of Comparative Example 1 was used instead of the polyol of Present Example 1.

### <Production Example 3>

The same procedure as that of Production Example 1 was performed, except that the polyol of Comparative Example 2 was used instead of the polyol of Present Example 1.

### <Production Example 4>

The same procedure as that of Production Example 1 was performed except that the polyol of Comparative Example 3 was used instead of the polyol of Present Example 1.

The amount of precipitation of allantoin was measured as follows. After measuring a weight of an empty container, 200 g of the liquid mixture of each of Production Example 1 to Production Example 4 was input into the container and then was stored therein in a refrigerated manner for 24 hours or 7 days. After the 24 hours or 7 days, a supernatant was removed therefrom, and the resulting mixture was dried in a thermostat to entirely remove any remaining supernatant, leaving only the precipitated portion. Then, the precipitated portion was dried for 12 hours, and then a weight thereof was measured. Thus, the amount of precipitation was derived as a difference value between the measured weight and the previously measured weight of the empty container.

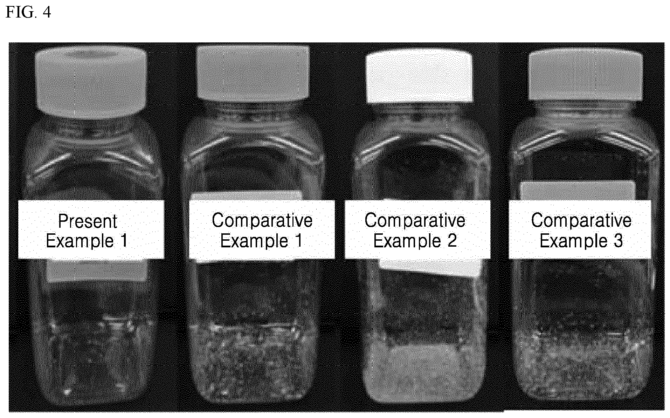

As a result, a relationship between the allantoin dispersion forces of the various polyols was as follows: Production Example 1 > Production Example 2 > Production Example 4 > Production Example 3. Therefore, it was identified that the dispersion force of the allantoin varied based on the type of polyol, and the dispersion force for allantoin was excellent when the polyol of Present Example 1 was used (FIG. 4 and Table 1). Therefore, when the polyol of Present Example 1 with a high content of (2R,3S)-2,3-butanediol was used, the precipitation amount and the precipitation rate of allantoin decreased, so that stable dispersion was realized.

**Table 1**

| | | Production Example 1 (use of Present Example 1) | Production Example 2 (use of Comparative Example 1) | Production Example 3 (use of Comparative Example 2) | Production Example 4 (use of Comparative Example 3) |
|---|---|---|---|---|---|
| Precipitation amount of allantoin (weight %) | After one day | 0 | 8 | 26 | 10 |
| | After 7 days | 29 | 38 | 48 | 39 |

### <2-2> Evaluation of dispersion force of allantoin in solubilized formulation

### <Production Example 5>

0.02 parts by weight of EDTA-2Na, 2 parts by weight of 1,2-hexanediol, and 0.5 parts by weight of allantoin were added to 100 parts by weight of purified water to prepare a water-based mixed solution. On the other hand, 0.1 parts by weight of fragrance, 0.3 parts by weight of PEG-60 hydrogenated castor oil, 5 parts by weight of 95% ethanol and 10 parts by weight of the polyol of Present Example 2 were mixed with each other to prepare a solubilized mixed solution. That is, the water-based mixed solution is composed of ultrapure water, EDTA-2Na, 1,2-hexanediol and allantoin, while the solubilized mixed solution is composed of fragrance, PEG-60 hydrogenated castor oil, ethanol and polyol.

The water-based mixed solution was stirred at room temperature for 3 minutes. On the other hand, the solubilized mixed solution was stirred was heating the same to 45 to 50 °C to make the same transparent. Then, the solubilized mixed solution was slowly added to the water-based mixed solution to prepare a mixture which in turn was stirred at room temperature for 2 minutes.

### <Production Example 6>

The same procedure as that of Production Example 5 was performed except that the polyol of Comparative Example 4 was used instead of the polyol of Present Example 2.

### <Production Example 7>

The same procedure as that of Production Example 5 was performed except that the polyol of Comparative Example 5 was used instead of the polyol of Present Example 2.

### <Production Example 8>

The same procedure as that of Production Example 5 was performed, except that the polyol of Comparative Example 6 was used instead of the polyol of Present Example 2.

The amount of precipitation of allantoin was measured as follows. After measuring a weight of an empty container, 200 g of the liquid mixture of each of Production Example 5 to Production Example 8 was input into the container and then was stored therein in a refrigerated manner for 24 hours or 7 days. After the 24 hours or 7 days, a supernatant was removed therefrom, and the resulting mixture was dried in a thermostat to entirely remove any remaining supernatant, leaving only the precipitated portion. Then, the precipitated portion was dried for 12 hours, and then a weight thereof was measured. Thus, the amount of precipitation was derived as a difference value between the measured weight and the previously measured weight of the empty container.

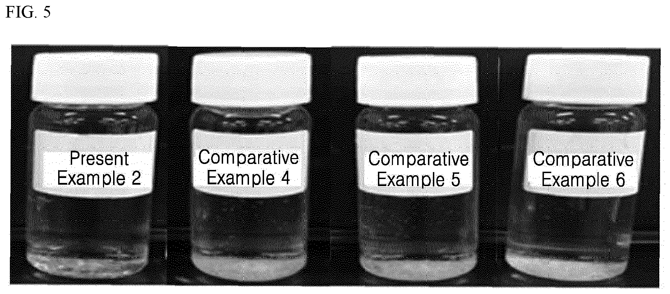

As a result, a relationship between the allantoin dispersion forces of the various polyols in the solubilized formulation was as follows: Production Example 5 > Production Examples 6 and 8 > Production Example 7. That is, after the storage for 7 days in the refrigerated manner, allantoin was precipitated in the solubilized formulation in each of Production Examples 6, 7 and 8 except Production Example 5. As a result, the allantoin dispersion force in the solubilized formulation was the best in Production Example 5. Therefore, it was identified that the dispersion force of the allantoin varied based on the type of polyol, and the dispersion force for allantoin was excellent when the polyol of Present Example 2 was used (FIG. 5 and Table 2). Therefore, when the polyol of Present Example 2 with a high content of (2R,3S)-2,3-butanediol was used, the precipitation amount and the precipitation rate of allantoin decreased, so that stable dispersion was realized.

**Table 2**

| | | Production Example 5 (use of Present Example 2) | Production Example 6 (use of Comparative Example 4) | Production Example 7 (use of Comparative Example 5) | Production Example 8 (use of Comparative Example 6) |
|---|---|---|---|---|---|
| Precipitation amount of allantoin (weight %) | After one day | 0 | 0 | 0 | 0 |
| | After 7 days | 0 | 7 | ~10 | 7 |

### <Experimental Example 3> Evaluation of dispersibility of vitamin C

Vitamin C is a water-soluble substance that dissolves well in water. However, when vitamin C is dissolved in water, it is oxidized and does not function properly. Therefore, in this experiment, vitamin C was applied to a polyol in oil (P/O) formulation as an anhydrous formulation, and an ivory-colored cream was prepared to evaluate dispersibility. It is more important to produce a stable cosmetic without discoloration, odor change, or phase separation rather than the problem of precipitation regarding vitamin C. Thus, factors for evaluating the dispersing power were set as soft spreadability, application uniformity, and emulsion stability.

### <3-1> Evaluation of dispersibility of vitamin C under non-heating condition

### <Production Example 9>

1 part by weight of EMERGENT JC 500B, 1.5 parts by weight of ABIL EM 90, and 2 parts by weight of KF 6017 were added to 100 parts by weight of ELASTOMER BASE to produce an oil-based mixed solution 1. On the other hand, 15 parts by weight of the polyol of Present Example 3 was used to prepare a water-based mixed solution 1. Further, 15 parts by weight of ascorbic acid ultra-fine powders was used to prepare a water-based mixed solution 2. Further, 45 parts by weight of KSG-16 was used to prepare an oil-based mixed solution 2.

The oil-based mixed solution 1 was stirred at room temperature. Then, the water-based mixed solution 2 was added to the water-based mixed solution 1 (polyol) at room temperature to produce a mixture which in turn was stirred. The mixture of the water-based mixed solutions 1 and 2 was slowly added to the oil-based mixed solution 1 under a stirring operation to produce a first P/O emulsion. The oil-based mixed solution 2 was added to the first P/O emulsion under a stirring operation to produce a second P/O emulsion in a homogeneous state. The second P/O emulsion was stored.

That is, a non-aqueous emulsified cosmetic composition containing no water was produced, and then the stability of the formulation thereof was evaluated to test the difference in the dispersion force depending on the type of the polyol. The cosmetic composition was produced by causing vitamin C to be absorbed into silicone powders (silicone elastomer) using a dispersion medium (polyol) free of water, and then mixing silicone gel (KSG-16) with the silicone powder in which the pure vitamin C is absorbed. The produced cosmetic composition had a creamy texture.

### <Production Example 10>

The same procedure as that of Production Example 9 was performed except that the polyol of Comparative Example 7 was used instead of the polyol of Present Example 3.

### <Production Example 11>

The same procedure as that of Production Example 9 was performed, except that the polyol of Comparative Example 8 was used instead of the polyol of Present Example 3.

### <Production Example 12>

The same procedure as that of Production Example 9 was performed, except that the polyol of Comparative Example 9 was used instead of the polyol of Present Example 3.

After storing the second P/O emulsion of each of Production Example 9 to Production Example 12 at room temperature for 1 day and 8 weeks, stability and feeling in use thereof were evaluated.

The evaluation of each item was performed according to a 10-point scale scheme as a sensory evaluation by ten cosmetic formulation experts (10 points: very good, 8 points: good, 6 points: average, 4 points: bad, 1 point: very bad).The dispersion stability was evaluated as follows: unstable: phase separation between the oil phase and the aqueous phase, discoloration and/or odor change, normal: the oil phase being slightly separated, and stable: no oil phase separation.

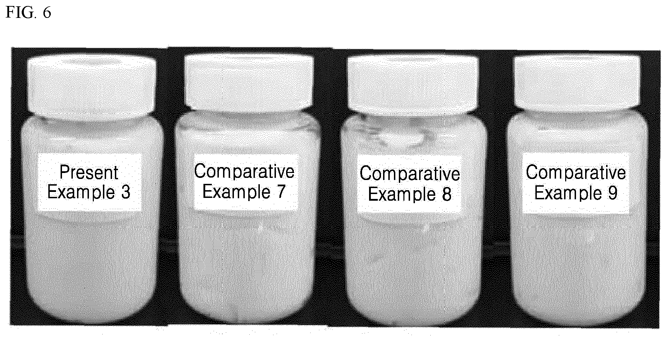

As a result, the Production Example 9 using Present Example 3 with a high content of (2R,3S)-2,3-butanediol had a smooth creamy phase and an ivory color and exhibited excellent smooth application, and uniformity of application. Further, in the Production Example 9, no phase separation between the oil phase and the aqueous phase was observed and a stable formulation without discoloration or order change was formed. The feeling in use and emulsion stability were evaluated after 8 weeks during the test. In Production Example 9 to which (2R,3S)-2,3-butanediol was applied, excellent spreadability, and uniformity of application were maintained, and there was no change in stability. In Production Example 12 to which 1,3-propanediol was applied, it was observed that a size of the particle became larger after 8 weeks such that a foreign body sensation occurred. Therefore, it was determined that when (2R,3S)-2,3-butanediol was used, the vitamin C was more stably dispersed and the feeling of foreign matter, that is, the feeling of particles was more significantly reduced, compared to when each of other polyols such as 1,3-butylene glycol, 1,3-propanediol, and (2R,3R)-2,3-butanediol was used (FIG. 6 and Table 3).

**Table 3**

| Evaluation items | After one day | | | | After 8 weeks | | | |
|---|---|---|---|---|---|---|---|---|
| | Productio n Example 9 (use of Present Example 3) | Production Example 10 (use of Comparativ e Example 7) | Production Example 11 (use of Comparativ e Example 8) | Production Example 12 (use of Comparativ e Example 9) | Productio n Example 9 (use of Present Example 3) | Production Example 10 (use of Comparativ e Example 7) | Production Example 11 (use of Comparativ e Example 8) | Production Example 12 (use of Comparativ e Example 9) |
| Soft application | 10 | 7 | 6 | 8 | 10 | 7 | 6 | 5 |
| Applicatio n uniformity | 9 | 4 | 4 | 6 | 9 | 4 | 4 | 4 |
| Emulsion stability | Stable | Stable | Stable | Stable | Stable | Stable | Stable | Stable |
| Dispersion force | Production Example 9 > Production Example 12 > Production Example 10 ≥ Production Example 11 | | | | Production Example 9 > Production Example 10 ≥ Production Example 11 > Production Example 12 | | | |

### <3-2> Evaluation of dispersibility of vitamin C under heating condition

### <Production Example 13>

1 part by weight of EMERGENT JC 500B, 1.5 parts by weight of ABIL EM 90, and 2 parts by weight of KF 6017 were added to 100 parts by weight of ELASTOMER BASE to produce an oil-based mixed solution 1. On the other hand, 15 parts by weight of the polyol of Present Example 4 was used to prepare a water-based mixed solution 1. Further. 15 parts by weight of ascorbic acid ultra-fine powder was used to prepare a water-based mixed solution 2. Further, 45 parts by weight of KSG-16 was used to prepare an oil-based mixed solution 2.

The oil-based mixed solution 1 was heated to 80 to 90°C while stirring the same. Then, the water-based mixed solution 2 was added to the water-based mixed solution 1 (polyol) to produce a mixture, and then the mixture was heated to 80 to 90°C. The mixture of the water-based mixed solution 1 and the water-based mixed solution 2 was slowly added to the heated oil-based mixed solution 1 under a stirring operation to produce a first P/O emulsion. The oil-based mixed solution 2 was added to the first P/O emulsion under a stirring operation to produce a second P/O emulsion in a homogeneous state. The second P/O emulsion was cooled to 30°C or lower and then was stored.

### <Production Example 14>

The same procedure as that of Production Example 13 was performed except that the polyol of Comparative Example 10 was used instead of the polyol of Present Example 4.

### <Production Example 15>

The same procedure as that of Production Example 13 was performed, except that the polyol of Comparative Example 11 was used instead of the polyol of Present Example 4.

### <Production Example 16>

The same procedure as that of Production Example 13 was performed, except that the polyol of Comparative Example 12 was used instead of the polyol of Present Example 4.

After storing the second P/O emulsion of each of Production Example 13 to Production Example 16 at room temperature for 1 day and 8 weeks, stability and feeling in use thereof were evaluated.

The evaluation of each item was performed according to a 10-point scale scheme as a sensory evaluation by ten cosmetic formulation experts (10 points: very good, 8 points: good, 6 points: average, 4 points: bad, 1 point: very bad).The dispersion stability was evaluated as follows: unstable: phase separation between the oil phase and the aqueous phase, discoloration and/or odor change, normal: the oil phase being slightly separated, and stable: no oil phase separation.

As a result, the Production Example 13 using Present Example 4 with a high content of (2R,3S)-2,3-butanediol had a smooth creamy phase and an ivory color and exhibited excellent smooth application, and uniformity of application. Further, in the Production Example 13, no phase separation between the oil phase and the aqueous phase was observed and a stable formulation without discoloration or order change was formed. The feeling in use and emulsion stability were evaluated after 8 weeks. In Production Example 13 to which (2R,3S)-2,3-butanediol was applied, excellent spreadability, and uniformity of application were maintained, and there was no change in stability. In Production Example 16 to which 1,3-propanediol was applied, it was observed that a size of the particle became larger after 8 weeks such that a foreign body sensation occurred. Therefore, it was determined that when (2R,3S)-2,3-butanediol was used, the vitamin C was more stably dispersed and the feeling of foreign matter, that is, the feeling of particles was more significantly reduced, compared to when each of other polyols such as 1,3-butylene glycol, 1,3-propanediol, and (2R,3R)-2,3-butanediol was used. On the contrary, it was determined that when each of 1,3-butylene glycol, 1,3-propanediol, and (2R,3R)-2,3-butanediol was applied, excess vitamin C was temporarily dissolved at a high temperature and then aggregation occurred in a non-uniformly dispersed portion and a size of the particles became large as the vitamin C was cooled down, thereby reducing the application uniformity.

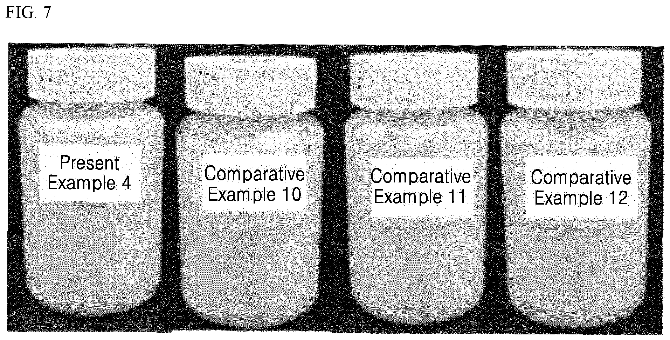

Further, in Experimental Example <3-1>, the formulation containing vitamin C was prepared under the non-heating condition, that is, under the room temperature condition, which was not the case in Experimental Example <3-2>. However, referring to the evaluation results, when (2R,3 S)-2,3-butanediol was applied to the production of the vitamin C-containing formulations, there was no significant difference between the properties under the heating/non-heating conditions. Therefore, it was determined that when (2R,3 S)-2,3-butanediol was applied to the production of the vitamin C-containing formulations, an effective dispersion force was achieved in the energy-saving process (FIG. 7 and Table 4).

Usually, when producing a non-aqueous emulsified cosmetic composition containing vitamin C, the pure vitamin C is dissolved in a heated solvent to prepare a solution which in turn is mixed with silicone powder to prepare a mixture which in turn was stirred to produce a water-based mixed solution. However, this process takes more time and energy because it goes through a heating process to apply heat and then a cooling process to cool the product. Therefore, in this Experimental Example 3, the heating process as performed in the industry and the non-heating process were performed. Then, it was evaluated whether the dispersion force was affected by the difference in the process, that is, whether the dispersion force under the heating process and that under the non-warming process were different from each other. As a result, it was identified that when the dispersion medium/dispersant composition using the 2,3-butanediol according to the present disclosure was used, the feeling in use and stability of the cosmetic formulation as produced under the heating condition were similar to those of the cosmetic formulation as produced under the non-heating condition. Therefore, it was determined that the 2,3-butanediol exhibited an effective dispersion force in the non-warm process, which saves the energy while having superior dispersion force compared to that of each of other polyols. Further, it was determined that the composition according to the present disclosure with a high content of (2R,3S)-2,3-butanediol exhibited significantly excellent dispersion force.

**Table 4**

| *Evaluatio n items | After one day | | | | After 8 weeks | | | |
|---|---|---|---|---|---|---|---|---|
| | Productio n Example 13 (use of Present Example 4) | Production Example 14 (use of Comparativ e Example 10) | Production Example 15 (use of Comparativ e Example 11) | Production Example 16 (use of Comparativ e Example 12) | Productio n Example 13 (use of Present Example 4) | Production Example 14 (use of Comparativ e Example 10) | Production Example 15 (use of Comparativ e Example 11) | Production Example 16 (use of Comparativ e Example 12) |
| Soft application | 10 | 7.5 | 7 | 8 | 10 | 7 | 6 | 5 |
| Application uniformity | 10 | 3 | 3 | 4 | 9 | 3 | 3 | 3 |
| Emulsion stability | Stable | Stable | Stable | Stable | Stable | Stable | Stable | Stable |
| Dispersion force | Production Example 13 > Production Example 16 > Production Example 14 ≥ Production Example 15 | | | | Production Example 13 > Production Example 14 ≥ Production Example 15 > Production Example 16 | | | |

### <Experimental Example 4> Evaluation of dispersibility for ceramide

Ceramide is an oil-soluble substance that is not soluble in water, precipitates easily during cosmetic production. It is difficult to stabilize ceramide due to gelling during the cosmetic production. In accordance with the present disclosure, the dispersion forces based on types of polyols were compared with each other using the characteristics of ceramide that precipitation thereof occurs well. The stability was evaluated at each of the room temperature, the refrigeration temperature, and the thermostat (45°C).

### <4-1> Evaluation of ceramide dispersion force

### <Production Example 17>

1 part by weight of ceramide was mixed with 100 parts by weight of the composition of Present Example 5, and the mixture was subjected to a dispersion step at room temperature, and was heated to 85 to 90 °C for dissolving and then was cooled to 30 °C or lower.

### <Production Example 18>

The same procedure as that of Production Example 17 was performed except that the polyol of Comparative Example 13 was used instead of the polyol of Present Example 5.

### <Production Example 19>

The same procedure as that of Production Example 17 was performed except that the polyol of Comparative Example 14 was used instead of the polyol of Present Example 5.

### <Production Example 20>

The same procedure as that of Production Example 17 was performed, except that the polyol of Comparative Example 15 was used instead of the polyol of Present Example 5.

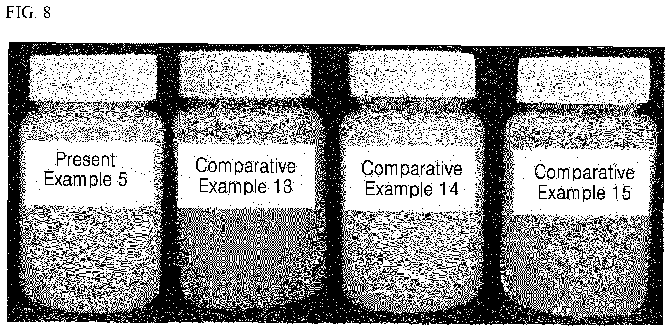

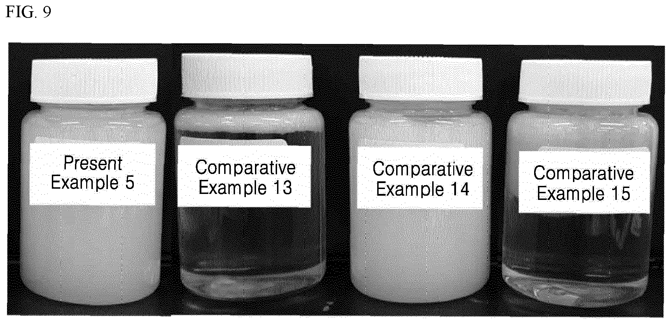

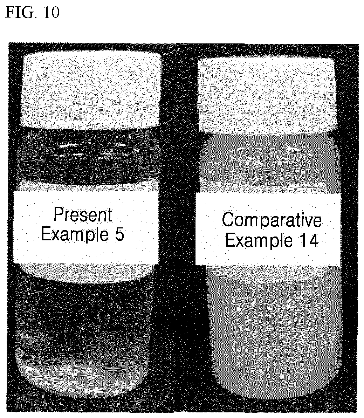

As a result, it was identified that the ceramide dispersion force was the best in Production Example 17 using Present Example 5 with a high content of (2R,3 S)-2,3-butanediol and Production Example 19 using 1,3-butylene glycol, while each of Production Example 18 using glycerin and Production Example 20 using 1,3-propanediol had low dispersion force of ceramide. That is, each of Production Examples 17 and 19 was completely dissolved and became transparent when being heated. However, when each of Production Examples 17 and 19 was cooled, ceramide was homogeneously precipitated to produce an opaque liquid (FIG. 8). The stability was evaluated after 4 weeks. In this regard, a viscosity of Production Example 19 to which 1,3-butylene glycol was applied increased to the extent that no flowability was observed. On the contrary, in Production Example 17 to which (2R,3S)-2,3-butanediol was applied, the flowability of the formulation was identified. On the other hand, when each of Production Example 18 to which glycerin was applied and Production Example 20 to which 1,3-propanediol was applied was heated, ceramide was not dissolved but was precipitated therein. The precipitated ceramide was separated from the polyol and floated to a top, and the separated state was maintained at room temperature for 4 weeks (FIG. 9). Additionally, based on a result of observation in a thermostat (45°C) for 4 weeks, Production Example 17 to which (2R,3S)-2,3-butanediol was applied was completely dissolved and thus became transparent (FIG. 10). Thus, no gelling was observed under all conditions. Therefore, it was identified that when the polyol of Present Example 5 with a high content of (2R,3 S)-2,3-butanediol was used, ceramide was stably dispersed. In particular, it was identified that polyol of Present Example 5 with a high content of (2R,3 S)-2,3-butanediol had excellent dispersing power in the thermostat (45 degrees Celsius) condition, such that the solution containing ceramide was completely dissolved and thus became transparent.

### <4-2> Evaluation of ceramide dispersion force in solubilized skin formulation

### <Production Example 21>

0.02 parts by weight of EDTA-2Na and 2 parts by weight of 1,2-hexanediol were added to 100 parts by weight of purified water to prepare a water-based mixed solution 1. On the other hand, 0.1 parts by weight of fragrance, 0.3 parts by weight of PEG-60 hydrogenated castor oil, and 5 parts by weight of DPG (Dipropylene Glycol) were mixed with each other to produce a solubilized mixed solution. Further, a water-based mixed solution 2 was prepared by mixing 10 parts by weight of polyol of Present Example 6 and 1 part by weight of ceramide with each other.

The water-based mixed solution 1 was stirred at room temperature for 2 minutes. On the other hand, the solubilized mixed solution was heated to 45 to 50°C while stirring the same to make the same transparent, and then was slowly added to the water-based mixed solution under a stirring operation. Then, the mixture was stirred at room temperature for 2 minutes. Then, the water-based mixed solution 2 was heated to 80 to 85 °C while stirring the same, and then the ceramide was added thereto. Then, the solution containing the ceramide was added to the mixture of the water-based mixed solution 1 and the solubilized mixed solution under a stirring operation. Then, a resulting mixture was stirred at room temperature for 3 minutes.

### <Production Example 22>

The same procedure as that of Production Example 21 was performed except that the polyol of Comparative Example 16 was used instead of the polyol of Present Example 6.

### <Production Example 23>

The same procedure as that of Production Example 21 was performed except that the polyol of Comparative Example 17 was used instead of the polyol of Present Example 6.

### <Production Example 24>

The same procedure as that of Production Example 21 was performed except that the polyol of Comparative Example 18 was used instead of the polyol of Present Example 6.

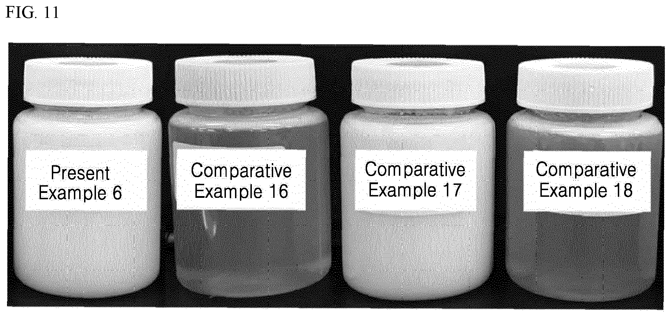

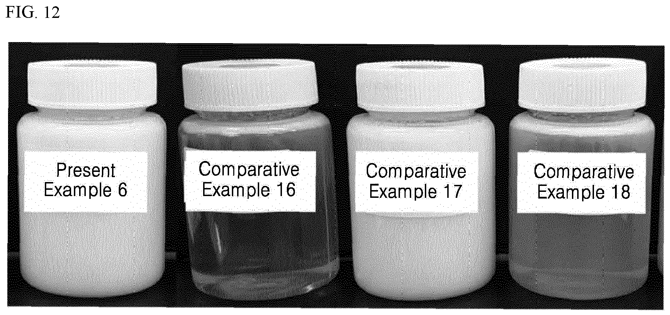

As a result, it was identified that the ceramide dispersion force was the best in each of Production Example 21 using Present Example 6 with a high content of (2R,3S)-2,3-butanediol and Production Example 23 using 1,3-butylene glycol, while each of Production Examples 22 and 24 exhibited low dispersion force of ceramide. That is, in Production Examples 21 and 23, ceramide was homogeneously dispersed. Further, based on a result of observation for 4 weeks, the same state was maintained at all of the room temperature, the refrigeration temperature (4°C), and the thermostat (45 °C) (FIG. 11 and FIG. 12). On the contrary, in each of Production Example 22 using glycerin and Production Example 24 using 1,3-propanediol, ceramide was precipitated and separated and floated to a top, and the separated state was maintained at the room temperature for 4 weeks. No gelling was observed under all conditions. Therefore, it was identified that when the polyol of Present Example 6 with a high content of (2R,3 S)-2,3-butanediol was used, ceramide was stably dispersed, and the dispersion force of ceramide was excellent under both the refrigeration temperature (4°C) condition, and the thermostat (45 °C) condition.

Further, an experiment for evaluating the dispersion force of ceramide was performed by applying the same to each of the solubilized essence formulation and the O/W emulsion formulation. As a result, it was identified that when (2R,3S)-2,3-butanediol was used, the dispersion force was excellent, and no gelling phenomenon was observed.

### INDUSTRIAL APPLICABILITY

The present disclosure relates to a dispersant composition comprising 2,3-butanediol and a cosmetic composition comprising the same. Further, the present disclosure relates to a dispersion medium composition comprising 2,3-butanediol and a cosmetic composition comprising the same.

## Claims

1. A dispersant composition comprising 2,3-butanediol.

2. The dispersant composition of claim 1, wherein 2,3-butanediol is (2R,3S)-2,3-butanediol.

3. The dispersant composition of claim 1, wherein the dispersant composition has a dispersion force of vitamin C, ceramide or allantoin higher than a dispersion force of vitamin C, ceramide or allantoin which (2R,3R)-2,3-butanediol or a 2,3-butanediol isomer mixture has, wherein in the 2,3-butanediol isomer mixture, a content of (2R,3R)-2,3-butanediol is higher than a content of 2,3-butanediol isomers other than (2R,3R)-2,3-butanediol.

4. The dispersant composition of claim 1, wherein 2,3-butanediol is a biologically based 2,3-butanediol obtained using microbial fermentation.

5. A cosmetic composition comprising the dispersant composition of one of claims 1 to 4.

6. The cosmetic composition of claim 5, wherein the cosmetic composition contains at least one selected from a group consisting of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients.

7. The cosmetic composition of claim 5, wherein the cosmetic composition is a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.

8. A dispersion medium composition comprising 2,3-butanediol.

9. The dispersion medium composition of claim 8, wherein 2,3-butanediol is (2R,3S)-2,3-butanediol.

10. The dispersion medium composition of claim 8, wherein the dispersion medium composition has a dispersion force of vitamin C, ceramide or allantoin higher than a dispersion force of vitamin C, ceramide or allantoin which (2R,3R)-2,3-butanediol or a 2,3-butanediol isomer mixture has, wherein in the 2,3-butanediol isomer mixture, a content of (2R,3R)-2,3-butanediol is higher than a content of 2,3-butanediol isomers other than (2R,3R)-2,3-butanediol.

11. The dispersion medium composition of claim 8, wherein 2,3-butanediol is a biologically based 2,3-butanediol obtained using microbial fermentation.

12. A cosmetic composition comprising the dispersion medium composition of one of claims 8 to 11.

13. The cosmetic composition of claim 12, wherein the cosmetic composition contains at least one selected from a group consisting of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients.

14. The cosmetic composition of claim 12, wherein the cosmetic composition is a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.

15. A method for producing a dispersant composition, the method comprising adding and mixing 2,3-butanediol to and with a solvent.

16. The method of claim 15, wherein 2,3-butanediol is (2R,3S)-2,3-butanediol.

17. The method of claim 15, wherein the dispersant composition has a dispersion force of vitamin C, ceramide or allantoin higher than a dispersion force of vitamin C, ceramide or allantoin which (2R,3R)-2,3-butanediol or a 2,3-butanediol isomer mixture has, wherein in the 2,3-butanediol isomer mixture, a content of (2R,3R)-2,3-butanediol is higher than a content of 2,3-butanediol isomers other than (2R,3R)-2,3-butanediol.

18. The method of claim 15, wherein 2,3-butanediol is a biologically based 2,3-butanediol obtained using microbial fermentation.

19. A method of producing a cosmetic composition, the method comprising adding and mixing the dispersant composition produced by the producing method of one of claims 15 to 18 to and with a cosmetic material.

20. The method of claim 19, wherein the cosmetic composition contains at least one selected from a group consisting of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients.

21. The method of claim 19, wherein the cosmetic composition is a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.

22. A method of producing a dispersion medium composition, the method comprising adding and mixing 2,3-butanediol to and with a solvent.

23. The method of claim 22, wherein 2,3-butanediol is (2R,3S)-2,3-butanediol.

24. The method of claim 22, wherein the dispersion medium composition has a dispersion force of vitamin C, ceramide or allantoin higher than a dispersion force of vitamin C, ceramide or allantoin which (2R,3R)-2,3-butanediol or a 2,3-butanediol isomer mixture has, wherein in the 2,3-butanediol isomer mixture, a content of (2R,3R)-2,3-butanediol is higher than a content of 2,3-butanediol isomers other than (2R,3R)-2,3-butanediol.

25. The method of claim 22, wherein 2,3-butanediol is a biologically based 2,3-butanediol obtained using microbial fermentation.

26. A method of producing a cosmetic composition, the method comprising adding and mixing the dispersion medium composition produced by the producing method of one of claims 22 to 25 to and with a cosmetic material.

27. The method of claim 26, wherein the cosmetic composition contains at least one selected from a group consisting of vitamin based raw materials including vitamin C, vitamin A, vitamin B, vitamin E, vitamin F, and vitamin H, moisturizing raw materials including allantoin and ceramide, whitening functional ingredients including arbutin and ethyl ascorbyl ether, wrinkle-suppressing functional ingredients including retinyl palmitate, adenosine, and polyethoxylated retinamide, functional ingredients for blocking UV rays including glyceryl FABA, drometrizole, benzophenone, and cinoxate, functional ingredients for reducing hair loss including biotin, L-menthol, and zinc pyrithione, anti-oxidation or discoloration prevention raw materials including ferulic acid and hexylresorcinol, exfoliating raw materials including salicylic acid and its salts and esters, and glycolic acid; functional protein and polymer raw materials including collagen and peptides, plant extracts, fragrances, pigments, pearls, powders, titanium dioxide, hair dedyeing or decoloring raw materials, anti-inflammatory ingredients, antioxidant ingredients, acne and atopic skin improvement ingredients.

28. The method of claim 26, wherein the cosmetic composition is a formulation selected from a group consisting of skin care products including softening lotion, astringent lotion, nutrient lotion, lotion, gel, cream, essence, eye essence, eye cream, nourishing cream, massage cream, clay-type pack, and mask pack, makeup products including lipstick, lip tint, lip gloss, lip pencil, eye shadow, foundation, powder, concealer, eyeliner, eye shadow, and mascara, cleansing products including eye remover, makeup remover, cleansing foam, cleansing cream, cleansing oil, cleansing water, hand sanitizer, hand wash, hand scrub, body wash, body scrub, shaving lotion, soap, and wet wipe, body care products including body lotion, body oil, body mist, body essence, hand cream, foot cream, and wax-type hair removal agent, baby cosmetics including baby lotion, baby cream, and diaper-induced rash prevention cream, sun care products including sun cream, sun stick, sun spray and artificial tanning products, hair products including shampoo, conditioner, hair conditioner cream, hair styling gel, foam, hair mousse, hair spray, styling lotion, styling cream, hair essence, hair dye, hair decoloring cream, and curl activator gel, nail care products including manicure, nail polish, cuticle remover, and cuticle cream, personal care products including toothpaste, mouthwash, mouth rinse, oral film, and gum, perfume, plant extract, deodorant and antiperspirant.
